# EUROPEAN PATENT APPLICATION

(11) **EP 4 659 771 A1**
(43) Date of publication of application: **10.12.2025**
(21) Application number: 25176859.4
(22) Date of filing: 16.05.2025
(51) Int. Cl.: A61L 27/20, A61L 27/50, A61L 27/52, A61L 27/58

(54) **INTERDENTAL PAPILLA RECONSTRUCTION METHOD USING HYALURONIC ACID**

(30) Priority: 05.06.2024 KR 20240073593
(71) Applicant: Novabio Co., Ltd., Gunpo-si, Gyeonggi-do 15850 (KR)
(72) Inventor: SEO, Hyun Bae, 0000 Seongnam-si, Gyeonggi-do (KR); BAE, Min Jun, 0000 Hwaseong-si, Gyeonggi-do (KR); HAN, Dong geun, 0000 Gyeongsan-si, Gyeongsangbuk-do (KR)
(74) Representative: Isarpatent

(57) **Abstract**

The disclosure relates to an interdental papilla reconstruction method using hyaluronic acid. One aspect of the disclosure, the interdental papilla reconstruction method, can conveniently reconstruct the lost or receded interdental papilla compared to conventional treatment methods. In addition, according to an aspect of the disclosure, the interdental papilla reconstruction method, the interdental papilla can be effectively reconstructed without pain or side effects. Further, the interdental papilla reconstruction method of the disclosure is highly useful as a rescue treatment for quickly resolving unexpected gingival recession or interdental papilla loss occurring during dental treatments.

## Description

### CROSS REFERENCE TO RELATED APPLICATION

The present application claims priority to Korean Patent Application No. 10-2024-0073593, filed on June 05, 2024, the entire contents of which are hereby incorporated by this reference.

### BACKGROUND OF THE INVENTION

### Field of the Invention

The disclosure relates to an interdental papilla reconstruction method using hyaluronic acid.

### Description of the Related Art

The interdental papilla is part of the free gingiva that occupies the space between two adjacent teeth and allows the free gingival margin to extend in a festoon-like pattern from the dental arch.

The interdental papilla firmly supports the teeth to help maintain their stability and prevent food debris and foreign objects from getting stuck between the teeth. In addition, the interdental papilla fills in between the teeth to provide a natural and balanced smile, thereby serving an aesthetic function.

The interdental papilla may be lost due to abnormal tooth shape, improperly shaped restorations, incorrect oral hygiene, and periodontal disease. When the interdental papilla is lost or receded, a so-called black triangle (black triangle syndrome or recession of the incisive papilla) occurs, which causes various functional and aesthetic problems as described above.

Therefore, up to now, in order to solve the problem of the loss of interdental papilla, gum regeneration surgery that involves transplanting gum tissue, resin filling that involves filling the space between teeth with resin, and prosthetic adjustments or orthodontic treatment that involves narrowing the space between teeth by using crowns, bridges, etc. have been used. However, the interdental papilla reconstruction method known to date has a complicated treatment procedure, causes pain, takes a long time to regenerate the interdental papilla, and requires expensive treatment costs. In addition, the method has inherent limitations, such as a minimal effect of interdental papilla reconstruction, leading to patients avoiding these treatments.

### Documents of Related Art

(Patent Document 0001) Korean Patent No. 10-1457231 (October 27, 2014)
(Patent Document 0002) Korean Patent Publication No. 10-2018-0020727 (February 28, 2018)

### SUMMARY OF THE INVENTION

In one aspect, the disclosure is directed to reconstructing lost or receded interdental papilla.

In one aspect, the disclosure is directed to eliminating a black triangle.

In one aspect, the disclosure is directed to reconstructing the interdental papilla in a convenient manner and to eliminate black triangles.

In one aspect, the disclosure is directed to providing an interdental papilla reconstruction method that has no side effects.

In one aspect, the disclosure is directed to providing an interdental papilla reconstruction composition that improves the pain experienced during administration (injection).

In one aspect, the disclosure is directed to reconstructing the interdental papilla in a simple and economical way compared to the conventional treatment method.

In one aspect, the disclosure is directed to increasing the soft tissue of the interdental papilla (increase in quantity and increase in volume).

In one aspect, the disclosure is directed to promoting an increase in the transverse (horizontal thickness) and longitudinal (vertical height) dimensions of the interdental papilla.

In one aspect, the disclosure is directed to promote the phenomenon of creeping attachment.

The objects to be achieved by the embodiments are not limited to the above-mentioned objects, but also include objects or effects that may be understood from the solutions or embodiments described below.

In order to achieve the aforementioned objects, in one aspect, the disclosure provides an interdental papilla reconstruction method.

In addition, in the aspect described above, the disclosure provides the interdental papilla reconstruction method, including a composition administration step of administering a composition including hyaluronic acid or a pharmaceutically acceptable salt thereof to gingiva, in which the composition includes a composition with a complex viscosity of 250 to 850 Pa·s.

In addition, in the aspect described above, the disclosure provides the interdental papilla reconstruction method in which the administration step includes administering the composition in an amount of 0.005 to 0.05 cc.

In addition, in the aspect described above, the disclosure provides the interdental papilla reconstruction method in which the administration step includes administering the composition 1 to 10 administrations, in an amount of 0.001 to 0.01 cc per administration.

In addition, in the aspect described above, the disclosure provides the interdental papilla reconstruction method in which the administration step includes administering the composition to the gingiva with an area of 0.1 to 4 mm².

In addition, in the aspect described above, the disclosure provides the interdental papilla reconstruction method in which the composition includes a gel formulation.

In addition, in the aspect described above, the disclosure provides the interdental papilla reconstruction method in which the method has an interdental papilla reconstruction rate expressed by Equation 1 below of 70% or more.

### [Equation 1]

Interdental papilla reconstruction rate(%)= 100 X Black triangle area after composition administration/black triangle area before composition administration.

In addition, in the aspect described above, the disclosure provides the interdental papilla reconstruction method in which the composition is administered using a needle of 25 to 35G.

In addition, in the aspect described above, the disclosure provides the interdental papilla reconstruction method in which the administration step includes administering the composition to a lower 1 to 3 mm site of an apex of an interdental papilla.

In addition, in the aspect described above, the disclosure provides the interdental papilla reconstruction method, which further includes a molding step.

In addition, in the aspect described above, the disclosure provides the interdental papilla reconstruction method in which the method is performed at intervals of 2 to 4 weeks.

In addition, in the aspect described above, the disclosure provides an interdental papilla reconstruction kit, including: a composition including hyaluronic acid or a pharmaceutically acceptable salt thereof; and a treatment manual describing the interdental papilla reconstruction method.

One aspect of the disclosure, the interdental papilla reconstruction method, can conveniently reconstruct the lost or receded interdental papilla compared to conventional treatment methods. In addition, according to an aspect of the disclosure, the interdental papilla reconstruction method, the interdental papilla can be effectively reconstructed without pain or side effects. Further, the interdental papilla reconstruction method of the disclosure is highly useful as a rescue treatment for quickly resolving unexpected gingival recession or interdental papilla loss occurring during dental treatments.

The various, beneficial advantages and effects of the disclosure are not limited to the above-mentioned contents and may be more easily understood during the process of describing the specific embodiments of the disclosure.

### BRIEF DESCRIPTION OF THE DRAWINGS

FIG. 1 illustrates the interdental papilla reconstruction rate (%) according to the complex viscosity of a hyaluronic acid gel included in a composition and time elapsed.
FIG. 2 is a diagram illustrating the interdental papilla reconstruction rate (%) according to the dose of composition and time elapsed.
FIG. 3 is a diagram illustrating the administration position and the administration area of the composition (the black-framed box marked with a black dot in the center indicates the administration position (dot) and the administration area).

### DETAILED DESCRIPTION OF THE INVENTION

The disclosure may be variously modified and may have various embodiments, and particular embodiments will be illustrated and described herein. However, the description of the embodiments is not intended to limit the disclosure to the particular embodiments, but it should be understood that the disclosure is to cover all modifications, equivalents and alternatives falling within the spirit and technical scope of the disclosure.

In addition, terms or words used in the specification and the claims of the disclosure should not be interpreted as being limited to a general or dictionary meaning and should be interpreted as a meaning and a concept which conform to the technical spirit of the disclosure based on a principle that an inventor can appropriately define a concept of a term in order to describe his/her own invention by the best method.

The terms including ordinal numbers such as 'second', 'first', and the like may be used to describe various constituent elements, but the constituent elements are not limited by the terms. These terms are used only to distinguish one constituent element from another constituent element. For example, a second component may be named a first component, and similarly, the first component may also be named the second component, without departing from the scope of the disclosure. The term "and/or" includes any and all combinations of a plurality of the related and listed items.

In addition, the terminology used in this application is used for the purpose of describing particular embodiments only and is not intended to limit the disclosure. Singular expressions include plural expressions unless clearly described as different meanings in the context. In this application, it should be understood the terms "comprises," "comprising," "includes," "including," "containing," "has," "having" or other variations thereof are inclusive and therefore specify the presence of stated features, integers, steps, operations, elements, components, or combinations thereof, but do not preclude the presence or addition of one or more other features, integers, steps, operations, elements, components, or combinations thereof.

Unless otherwise defined, all terms used herein, including technical or scientific terms, have the same meaning as commonly understood by those skilled in the art to which the disclosure pertains. The terms such as those defined in a commonly used dictionary should be interpreted as having meanings consistent with meanings in the context of related technologies and should not be interpreted as ideal or excessively formal meanings unless explicitly defined in this specification.

Hereinafter, the disclosure will be described in detail.

The disclosure, in one aspect, is a method for interdental papilla reconstruction.

The disclosure, in one aspect, is an interdental papilla reconstruction method that includes a composition administration step of administering a composition including hyaluronic acid or a pharmaceutically acceptable salt thereof to the gingiva, in which the composition includes a composition with a complex viscosity of 200 to 850 Pa·s.

The hyaluronic acid is a sulfur-free polymer belonging to the glycosaminoglycan family, which is widely distributed in mammalian tissues, has a non-branching structure, and is composed of regular repeats of a disaccharide consisting of glucuronic acid and N-acetylglucosamine.

The size of hyaluronic acid varies greatly, but it exceeds 1,000 kDa, and in some tissues, it reaches up to 3,000 kDa to 9,000 kDa daltons.

In the disclosure, hyaluronic acid may be used either as it is or may be used in various salt forms, such as sodium.

In this specification, the term "pharmaceutically acceptable salt" may include all salts of a compound that are physiologically tolerable to the subject or target organism. A pharmaceutically acceptable salt is preferably a formulation of the compound that does not cause severe irritation to the organism to which the compound is administered and does not impair the biological activity and properties of the compound. The pharmaceutically acceptable salt may include acid addition salts formed by acids that form non-toxic acid addition salts containing pharmaceutically acceptable anions, for example, inorganic acids such as hydrochloric acid, sulfuric acid, nitric acid, phosphoric acid, hydrobromic acid, and hydroiodic acid, organic carboxylic acids such as tartaric acid, formic acid, citric acid, acetic acid, trichloroacetic acid, trifluoroacetic acid, gluconic acid, benzoic acid, lactic acid, fumaric acid, maleic acid, and salicylic acid, and sulfonic acids such as methanesulfonic acid, ethanesulfonic acid, benzenesulfonic acid, and p-toluenesulfonic acid.

When the hyaluronic acid or pharmaceutically acceptable salt thereof has a molecular weight of less than 500 kDa, it may carry the potential to induce skin inflammation. On the other hand, when the hyaluronic acid or sodium salt thereof exceeds a molecular weight of 3,000 kDa, the molecular weight becomes too large, making it difficult to properly administer to the gingival connective tissue layer, which may be undesirable.

In one aspect, the molecular weight of the hyaluronic acid or pharmaceutically acceptable salt thereof may be 1,000 kDa to 3,000 kDa. Alternatively, the molecular weight of the hyaluronic acid or pharmaceutically acceptable salt thereof may be 1,300 kDa to 2,500 kDa, or 1,300 kDa to 2,300 kDa, or 1,500 kDa to 2,000 kDa.

In one aspect, the composition may include a composition with a complex viscosity of 200 to 850 Pa·s. Specifically, the complex viscosity of the composition may be 200 Pa·s or more, or 250 Pa·s or more, or 300 Pa·s or more, or 350 Pa·s or more, or 400 Pa·s or more, or 450 Pa·s or more, or 500 Pa·s or more, or 550 Pa·s or more, or 600 Pa·s or more, or 610 Pa·s or more, or 630 Pa·s or more, or 650 Pa·s or more, or 670 Pa·s or more, or 690 Pa·s or more, or 700 Pa·s or more, or 710 Pa·s or more, or 720 Pa·s or more, or 730 Pa·s or more, or 740 Pa·s or more, or 750 Pa·s or more, or 760 Pa·s or more, or 770 Pa·s or more, or 780 Pa·s or more, or 790 Pa·s or more, or 800 Pa·s or more, or may be 850 Pa·s or less, or 840 Pa·s or less, or 830 Pa·s or less, or 820 Pa·s or less, or 810 Pa·s or less, or 800 Pa·s or less, or 790 Pa·s or less, or 780 Pa·s or less, or 770 Pa·s or less, or 760 Pa·s or less, or 750 Pa·s or less, or 740 Pa·s or less, or 730 Pa·s or less, or 720 Pa·s or less, or 710 Pa·s or less, or 700 Pa·s or less, or 690 Pa·s or less, or 670 Pa·s or less, or 650 Pa·s or less, or 630 Pa·s or less, or 610 Pa·s or less, or 600 Pa·s or less, or 550 Pa·s or less, or 500 Pa·s or less, or 450 Pa·s or less, or 400 Pa·s or less, or 350 Pa·s or less, or 300 Pa·s or less, or 250 Pa·s or less.

In the aspect described above, the complex viscosity of the composition may be 200 to 850 Pa·s, or 300 to 850 Pa·s, or 400 to 850 Pa·s, or 500 to 850 Pa·s, or 500 to 800 Pa·s, or 500 to 750 Pa·s, or 550 to 750 Pa·s, or 570 to 750 Pa·s, or 570 to 730 Pa·s, or 590 to 730 Pa·s, or 600 to 710 Pa·s, or 601 to 700 Pa·s.

In this specification, the term "administration" refers to the introduction of individual components of a therapy for therapeutic treatment or condition improvement by any suitable method, and the route of administration may be through various route of oral or parenteral, as long as they can reach the target site (tissues, blood vessels, muscles, organs, body cavities, etc.). The term "administration" is used as a concept of encompassing all methods by which an individual component, composition, etc. may be delivered to a target tissue for therapeutic treatment or condition improvement, such as infusion, injection, dosing, instillation, oral administration, topical application, or local application.

In one aspect, the administration may involve administering the composition used in a single treatment either in a single administration or in multiple divided administrations. In one aspect, the composition may include an injectable composition, but is not limited thereto. For example, the composition may be formulated as an injection, suspension, or topical agent.

In one aspect, when the composition is administered by injection, the method of administration may be determined in consideration of factors such as the condition of the subject and the area of the administration site. The method of administration may include, but is not limited to: a linear injection method in which the composition is injected linearly while slowly withdrawing the needle after insertion; a point injection method in which the composition is administered in multiple small spots; a retrograde injection method in which the needle is deeply inserted and the composition is then injected while the needle is withdrawn; a cross-hatching injection method in which the linear injection method are made in intersecting vertical and horizontal directions to inject the composition in the form of a grid pattern; a fanning injection method in which the needle is inserted once and moved in multiple directions to inject the composition in a fan-shaped pattern; and a microcannula injection method in which the composition is administered using a blunt-tipped cannula instead of a needle.

The composition may have a formulation selected from the group consisting of an injection (a sterile aqueous solution), a suspension, and a topical agent, and may be in various parenteral formulations.

When the composition is formulated, it may include diluents or excipients such as fillers, extenders, binders, wetting agents, disintegrants, or surfactants that are used in the art. For example, when the composition is formulated as an injectable formulation, it may be prepared using an aqueous solvent such as physiological saline, or a non-aqueous solvent such as a vegetable oil, a higher fatty acid ester, an alcohol such as ethanol, benzyl alcohol, or glycerin. In addition, the composition may further include pharmaceutical carriers such as stabilizers for preventing deterioration of the formulation, such as ascorbic acid and EDTA, emulsifiers, crosslinking agents such as 1,4-butanediol diglycidyl ether (BDDE), buffering agents for pH adjustment, and preservatives for controlling microbial growth, such as cresol and benzyl alcohol (e.g., phenylmercuric nitrate, thimerosal, benzalkonium chloride, phenol, cresol, benzyl alcohol, etc.).

In one aspect, the injectable formulation may include hyaluronic acid or a pharmaceutically acceptable salt thereof, NaOH, BDDE, and the like. The amount of components other than hyaluronic acid or a pharmaceutically acceptable salt thereof, which are included in the injectable formulation, may be appropriately adjusted according to protocols known in the art for injectable formulations including hyaluronic acid or a pharmaceutically acceptable salt thereof.

In one aspect, the concentration of the hyaluronic acid or a pharmaceutically acceptable salt thereof may be 0.1 to 10% (1 mg/ml to 100 mg/ml), or 1 to 5% (10 mg/ml to 50 mg/ml), or 1 to 3% (10 mg/ml to 30 mg/ml), or 1.5 to 2.5% (15 mg/ml to 25 mg/ml).

In one aspect, when the composition is formulated as a suspension, injectable esters such as ethyl oleate, and vegetable oils such as propylene glycol or polyethylene glycol may be used.

In addition, in one aspect, when the composition is formulated as a topical agent, it may be formulated in a solid, semi-solid, or liquid form by adding inorganic or organic carriers, excipients, and diluents. For example, the topical agent may include a skin topical agent having a transdermal formulation such as an ointment, lotion, gel, cream, patch, spray, suspension, and emulsion.

In one aspect, when the viscosity of the composition is as described, it may demonstrate optimal diffusion within the interdental papilla and exhibit the reconstruction effect of the interdental papilla. For example, when the viscosity of the composition exceeds the above range, the excessively high viscosity may make it difficult to administer the composition into the interdental papilla, and may also result in a limitation in that the administered composition does not diffuse within the interdental papilla even after being administered.

In one aspect, the composition administration step may include administering the composition in an amount of 0.005 to 0.05 cc.

In one aspect, in the composition administration step, the amount of composition administered may be 0.005 cc or more, or 0.006 cc or more, or 0.007 cc or more, or 0.008 cc or more, or 0.009 cc or more, or 0.01 cc or more, or 0.011 cc or more, or 0.013 cc or more, or 0.015 cc or more, or 0.017 cc or more, or 0.019 cc or more, or 0.02 cc or more, or 0.021 cc or more, or 0.022 cc or more, or 0.023 cc or more, or 0.024 cc or more, or 0.025 cc or more, or 0.026 cc or more, or 0.027 cc or more, or 0.028 cc or more, or 0.029 cc or more, or 0.03 cc or more, or 0.035 cc or more, or 0.040 cc or more, or 0.045 cc or more, and may be 0.05 cc or less, or 0.049 cc or less, or 0.047 cc or less, or 0.045 cc or less, or 0.043 cc or less, or 0.041 cc or less, or 0.040 cc or less, or 0.039 cc or less, or 0.037 cc or less, or 0.035 cc or less, or 0.033 cc or less, or 0.031 cc or less, or 0.030 cc or less, or 0.029 cc or less, or 0.028 cc or less, or 0.027 cc or less, or 0.026 cc or less, or 0.025 cc or less, or 0.024 cc or less, or 0.023 cc or less, or 0.022 cc or less, or 0.021 cc or less, or 0.020 cc or less, or 0.019 cc or less, or 0.017 cc or less, or 0.015 cc or less, or 0.013 cc or less, or 0.011 cc or less, or 0.010 cc or less, or 0.009 cc or less, or 0.008 cc or less, or 0.007 cc or less, or 0.006 cc or less.

In one aspect, when the composition is administered in an amount smaller than the amount described above, it may be difficult to expect an interdental papilla reconstruction effect. When administered in excess, side effects such as the occurrence of tenderness, a foreign body sensation due to excessive increase in the volume of the interdental papilla site, and external leakage of the composition may occur.

In one aspect, the composition administration step may include administering the composition 1 to 10 administrations, in amounts of 0.001 to 0.01 cc per administration.

In one aspect, the amount of composition administered per administration (hereinafter referred to as a single administration dose) may be 0.001 to 0.01 cc. In one aspect, the amount of composition administered per administration may be 0.001 cc or more, or 0.0015 cc or more, or 0.002 cc or more, or 0.0025 cc or more, or 0.003 cc or more, or 0.0035 cc or more, or 0.004 cc or more, or 0.0041 cc or more, or 0.0042 cc or more, or 0.0043 cc or more, or 0.0044 cc or more, or 0.0045 cc or more, or 0.0046 cc or more, or 0.0047 cc or more, or 0.0048 cc or more, or 0.0049 cc or more, or 0.005 cc or more, or 0.0055 cc or more, or 0.006 cc or more, or 0.0065 cc or more, or 0.007 cc or more, or 0.0075 cc or more, or 0.008 cc or more, or 0.0085 cc or more, or 0.009 cc or more, or 0.0095 cc or more, and may be 0.01 cc or less, or 0.009 cc or less, or 0.0085 cc or less, or 0.0080 cc or less, or 0.0075 cc or less, or 0.0070 cc or less, or 0.0065 cc or less, or 0.0060 cc or less, or 0.0059 cc or less, or 0.0058 cc or less, or 0.0057 cc or less, or 0.0056 cc or less, or 0.0055 cc or less, or 0.0054 cc or less, or 0.0053 cc or less, or 0.0052 cc or less, or 0.0051 cc or less, or 0.005 cc or less, or 0.0045 cc or less, or 0.0040 cc or less, or 0.0035 cc or less, or 0.0030 cc or less, or 0.0025 cc or less.

In one aspect, the composition administration step may include administering the composition 1 to 10 administrations, when the composition is administered in the single administration dose.

In one aspect, the composition administration step may include administering the composition, when the composition is administered in the single administration dose, 1 administration or more, or 2 administrations or more, or 3 administrations or more, or 4 administrations or more, or 5 administrations or more, or 6 administrations or more, or 7 administrations or more, or 8 administrations or more, or 9 administrations or more, and may be administered with 10 administrations or less, or 9 administrations or less, or 8 administrations or less, or 7 administrations or less, or 6 administrations or less, or 5 administrations or less, or 4 administrations or less, or 3 administrations or less, or 2 administrations or less.

In one aspect, in the composition administration step, when the single administration dose is 0.001 to 0.01 cc, the number of administrations may be 1 to 10 administrations, or 2 to 8 administrations, or 3 to 7 administrations, or 4 to 6 administrations.

In addition, in one aspect, in the composition administration step, the single administration dose may be 0.003 to 0.007 cc, and in this case, the number of administrations may be 3 to 7 administrations, or 4 to 6 administrations, or 5 administrations.

Additionally, in one aspect, the composition administration step may include administering the composition to the gingiva with an area of 0.1 to 4 mm².

In one aspect, in the composition administration step, the area of the gingiva where the composition is administered may be 0.01 mm² or more, or 0.05 mm² or more, or 0.1 mm² or more, or 0.15 mm² or more, or 0.20 mm² or more, or 0.25 mm² or more, or 0.30 mm² or more, or 0.35 mm² or more, or 0.40 mm² or more, or 0.45 mm² or more, or 0.50 mm² or more, or 0.55 mm² or more, or 0.60 mm² or more, or 0.65 mm² or more, or 0.70 mm² or more, or 0.75 mm² or more, or 0.80 mm² or more, or 0.81 mm² or more, or 0.83 mm² or more, or 0.85 mm² or more, or 0.87 mm² or more, or 0.89 mm² or more, or 0.90 mm² or more, or 0.91 mm² or more, or 0.92 mm² or more, or 0.93 mm² or more, or 0.94 mm² or more, or 0.95 mm² or more, or 0.96 mm² or more, or 0.97 mm² or more, or 0.98 mm² or more, or 0.99 mm² or more, or 1.0 mm² or more, or 1.5 mm² or more, or 2.0 mm² or more, or 2.5 mm² or more, or 3.0 mm² or more, or 3.5 mm² or more, and may be 4.0 mm² or less, or 3.5 mm² or less, or 3.0 mm² or less, or 2.5 mm² or less, or 2.0 mm² or less, or 1.9 mm² or less, or 1.8 mm² or less, or 1.7 mm² or less, or 1.6 mm² or less, or 1.5 mm² or less, or 1.47 mm² or less, or 1.45 mm² or less, or 1.43 mm² or less, or 1.41 mm² or less, or 1.4 mm² or less, or 1.37 mm² or less, or 1.35 mm² or less, or 1.33 mm² or less, or 1.31 mm² or less, or 1.3 mm² or less, or 1.27 mm² or less, or 1.25 mm² or less, or 1.23 mm² or less, or 1.21 mm² or less, or 1.2 mm² or less, or 1.19 mm² or less, or 1.17 mm² or less, or 1.15 mm² or less, or 1.13 mm² or less, or 1.1 mm² or less, or 1.0 mm² or less, or 0.9 mm² or less, or 0.8 mm² or less, or 0.7 mm² or less, or 0.6 mm² or less, or 0.5 mm² or less, or 0.4 mm² or less, or 0.3 mm² or less, or 0.2 mm² or less, or 0.1 mm² or less, or 0.09 mm² or less, or 0.07 mm² or less, or 0.05 mm² or less, or 0.03 mm² or less, or 0.02 mm² or less.

In one aspect, the area of the gingiva where the composition is administered may be 0.5 to 3.0 mm², or 0.5 to 2.5 mm², or 0.5 to 2.0 mm², or 0.5 to 1.5 mm², or 0.7 to 1.3 mm², or 0.8 to 1.2 mm².

In one aspect, when the area of the gingiva where the composition is administered is within the above range, an excellent interdental papilla reconstruction rate may be achieved.

In one aspect, the composition may include a gel formulation.

Additionally, in one aspect, the method may have an interdental papilla reconstruction rate expressed by Equation 1 below of 70% or more. Interdental papilla reconstruction rate(%)= 100 × Black triangle area after composition administration/black triangle area before composition administration. (Black triangle: a triangular space formed between teeth when the interdental papilla is lost or receded.)

In one aspect, when the interdental papilla reconstruction method of the disclosure is applied, the interdental papilla reconstruction rate may be 70% or more, or 71 % or more, or 72% or more, or 73% or more, or 74% or more, or 75% or more, or 76% or more, or 77% or more, or 78% or more, or 79% or more, or 80% or more, or 85% or more, or 90% or more, or 95% or more.

In addition, in one aspect, the method may include the composition is administered using a needle of 25 to 35G. In one aspect, the composition may be administered using a 30G needle.

When the thickness of the injection needle is as described, the composition with the viscosity described above may be easily administered while minimizing the patient's pain.

In one aspect, the administration of the composition may be performed using a syringe equipped with an auto-injector.

Additionally, in one aspect, the composition administration step may include administering the composition to the lower 1 to 3 mm site of the apex of the interdental papilla.

In one aspect, the composition may be administered to the lower 2 to 3 mm site of the apex of the interdental papilla.

In one aspect, the method may further include a molding step.

In one aspect, the molding may refer to shaping or adjusting the site where the composition has been administered using hands, tools, or the like.

In one aspect, the molding step may be performed after the administration step.

In one aspect, through the molding, the administered composition may be adjusted to be properly positioned at the intended administration site. For example, through the molding, the administered composition may be adjusted to correct clumping or protrusion within the administration site, may be evenly distributed within the administration site, and may form a natural gingival line.

In one aspect, one embodiment of the molding may be expressed as a massage. The massage may refer to performing a massage such as applying gentle pressure using hands or the like to ensure that the composition injected into the administration site naturally settles within the administration site. In one aspect, through the massage, the composition may be made to flatten or elongate within the administration site.

Additionally, in one aspect, the interdental papilla reconstruction method may be performed at intervals of 2 to 4 weeks.

In addition, the interdental papilla reconstruction method may further include actions generally known to those skilled in the art for the reconstruction of the interdental papilla or for dental treatments. For example, it may include oral disinfection, anesthesia, compresses, massages, and the like.

Additionally, the disclosure relates to an interdental papilla reconstruction kit that includes a composition including hyaluronic acid or a pharmaceutically acceptable salt thereof and a treatment manual that describes the interdental papilla reconstruction method.

The manual may include information on the complex viscosity of the composition including hyaluronic acid or a pharmaceutically acceptable salt thereof, the administration unit area, the single administration dose, the number of administrations, and other precautions.

In one aspect, the composition including hyaluronic acid or a pharmaceutically acceptable salt thereof in the kit may be filled in a vessel or container. In one aspect, the hyaluronic acid or a pharmaceutically acceptable salt thereof may be filled in solid form (e.g., freeze-dried powder) or in liquid form, but is not limited thereto.

Additionally, in one aspect, the composition may be filled in a container according to the individual components contained in the composition. The kit may further include, in addition to the composition, anesthetics, anti-inflammatory agents, pH regulators, and the like. These may be individually filled in separate containers from the container in which the composition is filled.

Additionally, the kit may include an administration device for delivering the composition to the administration site. In one aspect, the administration device may be a syringe, but is not limited thereto.

The following examples will provide a detailed description of the disclosure. These examples are provided merely to illustrate the disclosure and the scope of the disclosure is not limited by these examples.

### [Experimental example 1] Evaluation of interdental papilla reconstruction rate according to complex viscosity of hyaluronic acid gel

Using a 30G needle (syringe) equipped with an auto-injector, the hyaluronic acid gel listed in Table 1 was administered in 5 administrations (total of 0.025 cc), with 0.005 cc per administration, to the 2-3 mm site of area of the apex of the interdental papilla of the test subject. The interdental papilla reconstruction rate was then evaluated according to time elapsed.

**TABLE 1**

| Test No. | Complex viscosity (Pa.s) |
|---|---|
| 1 | 1 to 79 |
| 2 | 80 to 199 |
| 3 | 200 to 399 |
| 4 | 400 to 449 |
| 5 | 500 to 600 |
| 6 | 601 to 700 |
| 7 | 849 to 900 |

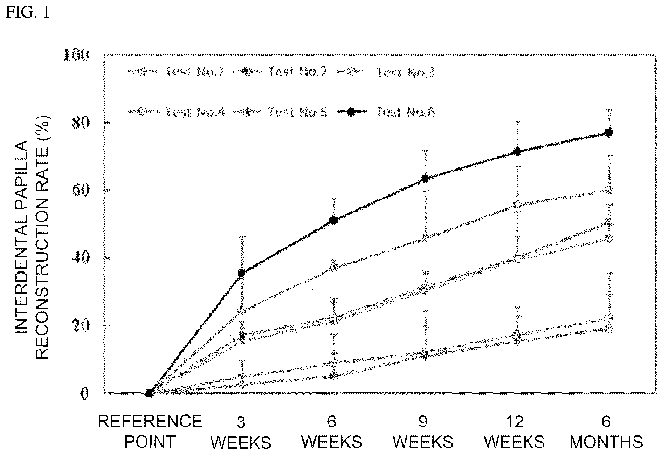

As a result, Test Nos. 1 to 6 showed an increase in the interdental papilla reconstruction rate in proportion to the time elapsed after administration. Additionally, it was confirmed that Test Nos. 1 to 6 showed an increase in the interdental papilla reconstruction rate in proportion to the complex viscosity (see FIG. 1).

Meanwhile, when the complex viscosity exceeds 700, as in Test No. 7, the complex viscosity becomes excessively high, making it difficult to administer into the interdental papilla, and thus it was not suitable for interdental papilla reconstruction.

### [Experimental example 2] Evaluation of interdental papilla reconstruction rate according to dose of hyaluronic acid gel

Test No. 6, which showed the best interdental papilla reconstruction rate in Experimental example 1, was injected into the apex of the interdental papilla using a syringe with a 30G needle equipped with an auto-injector.

To evaluate the interdental papilla reconstruction rate according to the dose of hyaluronic acid gel, the total doses were set at 0.005 cc, 0.01 cc, 0.025 cc, 0.05 cc, 0.1 cc, and 0.2 cc, and each was administered in 5 divided administrations (e.g., in case of a total dose of 0.005 cc, 0.001 cc per administration was administered in 5 administrations).

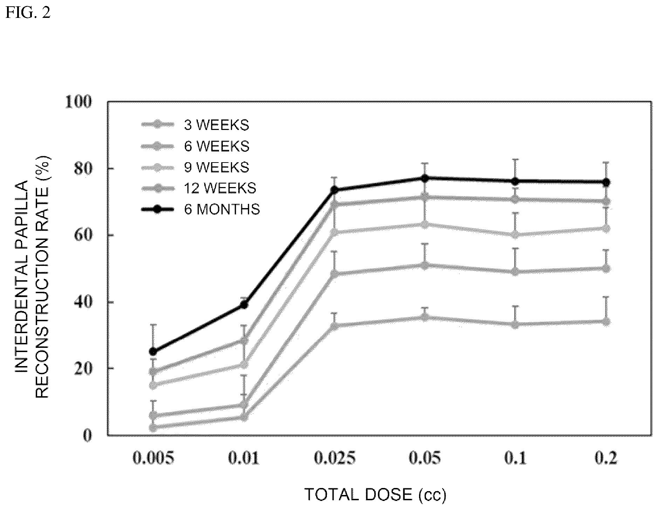

As a result, it was confirmed that the interdental papilla reconstruction rate increased in proportion to the total dose. Additionally, when the total dose exceeded 0.025 cc, the increase in the interdental papilla reconstruction rate was minimal, and there was a potential for an increased risk of side effects (see FIG. 2).

Thus, it was confirmed that the total dose of hyaluronic acid gel for maximizing the interdental papilla reconstruction rate is 0.025 cc.

### [Experimental Example 3] Evaluation of interdental papilla reconstruction rate (%) according to single administration dose per administration unit area

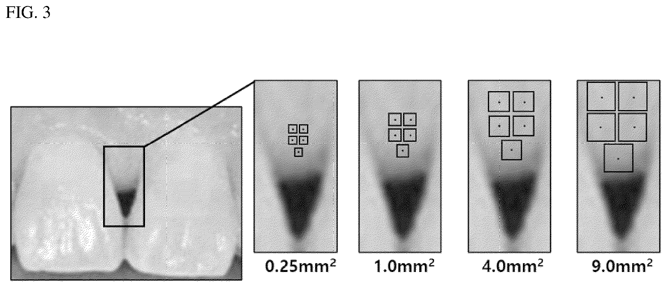

The administration areas for the hyaluronic acid gel were set at 0.25 mm², 1.0 mm², 4.0 mm², and 9.0 mm², with the dose per each area set at 0.001 cc, 0.002 cc, and 0.005 cc. The hyaluronic acid gel was administered 5 administrations for each area using a syringe with a 30G needle equipped with an auto-injector. The injection sites (administration sites) may be found in FIG. 3.

As a result, as shown in Table 2 below, the best interdental papilla reconstruction rate was confirmed when the single administration dose per administration unit area was 0.005 cc. In addition, the most improved interdental papilla reconstruction rate was confirmed when the administration area was 1 mm².

**TABLE 2**

| Administration unit area (mm2) | Single administration dose (cc) | Interdental papilla reconstruction rate(%) |
|---|---|---|
| 0.25 | 0.001 | 18.94 |
| | 0.002 | 28.07 |
| | 0.005 | 69.35 |
| 1.0 | 0.001 | 33.21 |
| | 0.002 | 41.38 |
| | 0.005 | 77.18 |
| 4.0 | 0.001 | 10.36 |
| | 0.002 | 17.94 |
| | 0.005 | 55.12 |
| 9.0 | 0.001 | 6.97 |
| | 0.002 | 12.19 |
| | 0.005 | 36.61 |

While the embodiments have been described above, the embodiments are just illustrative and not intended to limit the disclosure. It can be appreciated by those skilled in the art that various modifications and applications, which are not described above, may be made to this embodiment without departing from the intrinsic features of this embodiment. For example, the respective constituent elements specifically described in the embodiments may be modified and then carried out. Further, it should be interpreted that the differences related to the modifications and alterations are included in the scope of the disclosure defined by the appended claims.

## Claims

1. A composition including hyaluronic acid or a pharmaceutically acceptable salt thereof to gingiva,
wherein the composition comprises a composition with a complex viscosity of 250 to 850 Pa·s. for use in an interdental papilla reconstruction method, the comprising:
an administration step of administering said composition.

2. The composition for use in an interdental papilla reconstruction method of claim 1, wherein the administration step comprises administering the composition in an amount of 0.005 to 0.05 cc.

3. The composition for use in an interdental papilla reconstruction method of claim 1, wherein the administration step comprises administering the composition 1 to 10 administrations, in an amount of 0.001 to 0.01 cc per administration.

4. The composition for use in an interdental papilla reconstruction method of claim 1, wherein the administration step comprises administering the composition to the gingiva with an area of 0.1 to 4 mm².

5. The composition for use in an interdental papilla reconstruction method of claim 1, wherein the composition includes a gel formulation.

6. The composition for use in an interdental papilla reconstruction method of claim 1, wherein the method has an interdental papilla reconstruction rate expressed by Equation 1 below of 70% or more. Interdental papilla reconstruction rate(%)= 100 × Black triangle area after composition administration/black triangle area before composition administration.

7. The composition for use in an interdental papilla reconstruction method of claim 1, wherein the composition is administered using a needle of 25 to 35G.

8. The composition for use in an interdental papilla reconstruction method of claim 1, wherein the administration step comprises administering the composition to a lower 1 to 3 mm site of an apex of the interdental papilla.

9. The composition for use in an interdental papilla reconstruction method of claim 1, further comprising: a molding step.

10. The composition for use in an interdental papilla reconstruction method of claim 1, wherein the method is performed at intervals of 2 to 4 weeks.

11. An interdental papilla reconstruction kit, comprising:
a composition including hyaluronic acid or a pharmaceutically acceptable salt thereof; and
a treatment manual describing the interdental papilla reconstruction method according to any one of claims 1 to 10.
